# EUROPÄISCHE PATENTSCHRIFT

(11) **EP 1 300 388 B1**
(45) Veröffentlichungstag und Bekanntmachung des Hinweises auf die Patenterteilung: **07.12.2005**
(21) Anmeldenummer: 02018325.7
(22) Anmeldetag: 14.08.2002
(51) Int. Cl.: C07C 67/08, C07C 69/80

(54) **Verfahren zur Herstellung von Estern di- oder mehrbasiger Karbonsäuren**
Process for the production of di- or polycarboxylic acid esters
Procédé de préparation d'esters d'acides di- ou polycarboxyliques

(30) Priorität: 06.10.2001 DE 10149350
(43) Veröffentlichungstag der Anmeldung: 09.04.2003
(73) Patentinhaber: Oxeno Olefinchemie GmbH, 45772 Marl (DE)
(72) Erfinder: Ernst, Uwe, 45770 Marl (DE); Gubisch, Dietmar, Dr., 45772 Marl (DE)

(56) Entgegenhaltungen:
- DE-A- 2 112 492

## Beschreibung

Die Erfindung betrifft ein diskontinuierliches Verfahren zur Herstellung von Carbonsäureestern durch Umsetzung von di- oder mehrbasischen Carbonsäuren oder deren Anhydride mit Alkoholen.

Ester aus mehrbasischen Carbonsäuren, wie beispielsweise Phthalsäure, Adipinsäure, Sebacinsäure, Maleinsäure und Alkoholen finden weite Anwendung in Lackharzen, als Bestandteile von Anstrichmitteln und insbesondere als Weichmacher für Kunststoffe.

Es ist bekannt, Carbonsäureester durch Umsetzung von Carbonsäuren mit Alkoholen herzustellen. Diese Reaktion kann autokatalytisch oder katalytisch, beispielsweise durch Bronstedt- oder Lewissäuren, durchgeführt werden. Ganz gleich welche Art der Katalyse gewählt wird, es entsteht immer ein temperaturabhängiges Gleichgewicht zwischen den Einsatzstoffen (Carbonsäure und Alkohol) und den Produkten (Ester und Wasser). Um das Gleichgewicht zu Gunsten des Esters zu verschieben, wird bei vielen Veresterungen ein Schleppmittel eingesetzt, mit dessen Hilfe das Reaktionswasser aus dem Ansatz entfernt wird. Wenn einer der Einsatzstoffe (Alkohol oder Carbonsäure) niedriger siedet als der gebildete Ester und mit Wasser eine Mischungslücke bildet, kann dieses Edukt als Schleppmittel verwendet und nach Abtrennung vom Wasser wieder in den Ansatz zurückgeführt werden. Bei der Veresterung von zwei oder mehrbasigen Säuren ist in der Regel der eingesetzte Alkohol das Schleppmittel.

Für viele Anwendungszwecke müssen durch Veresterung einer Carbonsäure hergestellte Ester eine niedrige Säurezahl besitzen, d. h. die Umsetzung der Carbonsäure sollte praktisch quantitativ erfolgen. Anderenfalls wird die Ausbeute gemindert und die Säure muss, beispielsweise durch Neutralisation, abgetrennt werden. Dies ist aufwendig und kann zu Nebenprodukten führen, die entsorgt werden müssen. Um einen möglichst hohen Umsatz der Carbonsäure zu erhalten, werden Veresterungen in der Regel mit einem Alkoholüberschuss durchgeführt.
Als Veresterungskatalysatoren können Säuren, wie beispielsweise Schwefelsäure oder p-Toluolsulfonsäure, oder Metalle und deren Verbindungen eingesetzt werden. Geeignet sind z. B. Zinn, Titan, Zirconium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität erst bei Temperaturen oberhalb 180 °C erreichen. Sie werden jedoch in der Technik bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirconiumester wie Tetrabutylzirconat.

Nach der erfolgten Veresterung müssen der überschüssige Einsatzalkohol, Säurereste, der Katalysator und/oder seine Folgeprodukte sowie sonstige Nebenprodukte vom gewünschten Carbonsäureester getrennt werden. Die Wirtschaftlichkeit eines Veresterungsverfahren ist nur dann gut, wenn sowohl der Aufwand als auch die Zeit für die Stofftrennung gering sind.

Aus der Literatur sind verschiedene Veresterungsverfahren bzw. Aufarbeitungsverfahren für die so erhaltenen Rohestergemische bekannt.

In DE 19 45 359 wird ein Aufarbeitungsverfahren für Rohestergemische, die durch Umsetzung von Dicarbonsäuren mit 4 bis 12 C-Atomen mit Alkoholen mit 4 bis 16 C-Atomen in Gegenwart metallischer Katalysatoren erhalten wurden, beschrieben, das durch die Abfolge nachstehender Verfahrensschritte gekennzeichnet ist:
a) Die Restsäure im Rohestergemisch wird mit alkalischen Stoffen neutralisiert.
b) Der nicht umgesetzte Alkohol wird durch Wasserdampf entfernt.
c) Das Produkt wird auf Temperaturen abgekühlt, die unter dem Siedepunkt des Wassers beim jeweiligen Druck liegen.
d) Es werden mindestens 0,5 Massen-% Wasser, bezogen auf das aufzuarbeitende Produkt, zugesetzt.
e) Das Gemisch aus Wasser und aufzuarbeitendem Produkt wird mindestens 15 Minuten bei Temperaturen, die unter der Siedetemperatur des Wassers beim jeweiligen Druck liegen, intensiv gerührt.
f) Das zugesetzte Wasser wird durch Vakuumdestillation entfernt.
g) Der Ester wird filtriert.

In DE 26 12 355 ist ein Aufarbeitungsverfahren für Rohestergemische aus der Veresterung von Phthalsäureanhydrid oder Adipinsäure mit C₆-C₁₃-Alkoholen offengelegt, das folgende Verfahrensschritte umfasst:
a) Das heiße Veresterungsgemisch wird auf Temperaturen unter 100 °C abgekühlt.
b) Das abgekühlte Gemisch wird im Temperaturbereich von 80 bis 100 °C mit wässriger Lauge neutralisiert.
c) Die organische Phase wird durch Dekantieren und mit Wasser gewaschen.
d) Der größte Teil des nicht umgesetzten Alkohols wird abdestilliert.
e) Der Ansatz wird mit Wasserdampf gestrippt.
f) Das Estergemisch wird getrocknet.

Die Neutralisation der abgekühlten Reaktionsmischung erfolgt durch Zugabe von verdünnten Alkalilösungen (0,1 - 5 Gew.-%). Es sind daher recht große Volumina an Base erforderlich (5 - 20 Gew.-%, bezogen auf den Rohester), so dass zur Abtrennung der wässrigen Phase erhebliche Flüssigkeitsmengen bewegt werden müssen. Nach der Neutralisation und Wasserwäsche muss der Ansatz wieder aufgeheizt werden (Zeit- und Energieverlust).

Ein weiteres Aufarbeitungsverfahren für ein Rohestergemisch, erhalten durch Veresterung einer Carbonsäure mit einem Alkohol in Gegenwart einer metallorganischen Verbindung, wird in US 4 304 925 beschrieben. Die Aufarbeitung besteht aus folgenden Verfahrensschritten:
a) Das Rohestergemisch wird auf Temperaturen von unter 100 °C abgekühlt.
b) Zur Hydrolyse der metallorganischen Verbindungen wird der Ansatz mit Wasser versetzt und im Temperaturbereich von 60 bis 98 °C 30 bis 120 Minuten lang gerührt.
c) Zur Neutralisation der Säurereste wird eine Base (Alkalihydoxid, -carbonat oder hydrogencarbonat) zugesetzt und weitere 30 Minuten lang gerührt.
d) Die Wasserphase wird abgetrennt und die verbleibende organische Phase mit Wasser gewaschen.
e) Durch Wasserdampfdestillation im Vakuum wird der Einsatzalkohol abgetrieben.
f) Nach Zugabe eines Filterhilfsmittel wird der Ansatz filtriert.

US 5 434 294 beschreibt ein Verfahren zur Herstellung von Carbonsäureestern durch Umsetzung von Carbonsäuren mit Alkoholen, bei dem als Katalysator Titanorganische Verbindungen verwendet werden. Die Aufarbeitung des Rohgemisches zum Reinester umfasst folgende Verfahrensschritte:
a) Das Veresterungsgemisch wird auf 80 bis 150 °C abgekühlt.
b) Zur Neutralisation der Säurereste wird eine wässrige Base zugesetzt und 30 Minuten gerührt.
c) Der feuchte neutralisierte Reaktionsansatz wird durch eine Schicht Absorptionsmittel filtriert.
d) Der Einsatzalkohol wird durch Destillation z. B. in einem Dünnschichtverdampfer abgetrennt.

In WO 97/11048 wird eine Aufarbeitungsvorschrift für Veresterungsgemische, hergestellt aus Carbonsäuren und Alkoholen unter Verwendung von organischen Titanverbindungen als Katalysator, offenbart. Die Gewinnung des reinen Esters besteht aus folgenden Stufen:
a) Das Veresterungsgemisch mit einer Säurezahl von 0,56 mg KOH/g wird bei 220 °C durch Zugabe von 10 %iger wässriger Natriumhydrogencarbonat-Lösung neutralisiert und mit einem Schleppmittel wie Toluol versetzt.
b) Es wird auf 90 °C abgekühlt und 2 % Wasser, bezogen auf das Veresterungsgemisch, zugesetzt und gerührt.
c) Wasser, Ausgangsalkohol und sonstige Leichtsieder werden im Vakuum abdestilliert.
d) Der Destillationsrückstand wird filtriert.

In der Produktionsinformation der Hüls AG, "Alkyltitanate als Veresterungskatalysatoren zur Herstellung von Esterweichmachern", 606/93, (September 1993) wird u. a. eine Aufarbeitungsvariante mit folgenden Verfahrensschritte beschrieben:
a) Nach Erreichen einer Säurezahl von 1 wird der größte Teil des überschüssigen Alkohols durch Vermindern des Drucks bis auf 15 mbar abdestilliert.
b) Gegebenenfalls werden die Säurereste durch Zugabe von 0,1 % Natriumhydrogencarbonat, bezogen auf das Veresterungsgemisch neutralisiert.
c) Der Ansatz wird bei 150 bis 170 °C und bei einem Vakuum von 10 bis 15 mbar einer Wasserdampfdestillation unterworfen.
d) Nach dem Abkühlen auf 120 bis 100 °C und Zugabe eines Filterhilfsmittel und gegebenenfalls Aktivkohle wird filtriert.

In DE 197 21 347 C2 wird ein Verfahren zur Herstellung von Carbonsäureestern, hergestellt durch Umsetzung von Carbonsäuren mit Alkoholen in Gegenwart organischer Titan- oder Zirkoniumkatalysatoren, beschrieben. Darin ist die Aufarbeitung des rohen Veresterungsgemisches durch folgende Verfahrensschritte gekennzeichnet:
a) Säurereste werden durch Zugabe von Lauge bei der Veresterungsendtemperatur neutralisiert.
b) Der überschüssige Alkohol wird mit Wasserdampf abgetrieben.
c) Der Ansatz wird durch Einleiten eines Inertgases getrocknet.
d) Der Ansatz wird, gegebenenfalls nach Zusatz eines Filterhilfsmittel, filtriert.

In DE 100 43 545 wird ein diskontinuierliches Veresterungsverfahren beschrieben, bei dem das Reaktionswasser durch azeotrope Destillation mit Alkohol aus der Reaktion entfernt wird. Der auf diese Weise ebenfalls entfernte Alkohol wird der Reaktion nach Abtrennung des Wassers im Dekanter wieder zugeführt, so dass das Gleichgewicht der Veresterungsreaktion auf die Produktseite verschoben wird. Auch hier wird nach der Veresterung durch Zugabe von Basen neutralisiert, wobei dies vor oder nach Abtrennung des Alkohols durch Vakuumdestillation erfolgen kann.

Diese Verfahren sind im Hinblick auf die Abwasserbelastung, den Energieverbrauch und Zeitaufwand für die Aufarbeitung des Rohestergemisches verbesserungsfähig.

Es wurde gefunden, dass die Taktzeit und der Energieaufwand für die Aufarbeitung eines Veresterungsgemisch, entstanden durch Umsetzung einer Carbonsäure oder deren Anhydrid mit einem Alkohol in Gegenwart einer organischen Titan- oder Zirconiumverbindung gesenkt werden kann, wenn der größte Teil des überschüssigen Alkohols aus dem Reaktionsgemisch abdestilliert, der Ansatz unter Absenkung der Temperatur einer Wasserdampfdestillation unterworfen und gleichzeitig wässrige Base zudosiert wird.

Gegenstand der vorliegenden Erfindung ist daher ein Verfahren zur Herstellung von Carbonsäureestern durch Reaktion von Di- oder Polycarbonsäuren oder deren Anhydride mit Alkoholen, wobei der überschüssige Alkohol nach der Veresterungsreaktion entfernt, der so erhaltene Rohester durch Basenzugabe neutralisiert und anschließend filtriert wird, wobei der Alkohol durch mindestens eine Wasserdampfdestillation abgetrennt und die Basenzugabe während der Wasserdampfdestillation erfolgt.

Die Abtrennung des Alkohols erfolgt bevorzugt durch eine Vakuumdestillation und eine anschließende Wasserdampfdestillation.

Das erfindungsgemäße Verfahren hat den Vorteil, dass im Vergleich zu den bekannten diskontinuierlichen Verfahren die Aufarbeitungszeit verkürzt, die Ausbeute erhöht und die Menge an Filtersalzen verringert wird, wodurch sich eine höhere Wirtschaftlichkeit ergibt.

Im erfindungsgemäßen Verfahren werden als Säurekomponente Di- und Polycarbonsäuren sowie deren Anhydride eingesetzt. Bei mehrbasigen Carbonsäuren können auch teilweise anhydridisierte Verbindungen eingesetzt werden. Ebenso ist es möglich, Gemische aus Carbonsäuren und Anhydriden zu verwenden. Die Säuren können aliphatisch, carbocyclisch, heterocyclisch, gesättigt oder ungesättigt sowie aromatisch sein. Aliphatische Carbonsäuren besitzen mindestens 4 C-Atome. Beispiele für aliphatische Carbonsäuren bzw. deren Anhydride sind: Bernsteinsäure, Bernsteinsäureanhydrid, Korksäure, Azelainsäure, Decandisäure, Dodecandisäure, Brassylsäure. Beispiele für carbocyclische Verbindungen sind: Hexahydrophthalsäureanhydrid, Hexahydrophthalsäure, Cyclohexan-1.4-dicarbonsäure, Cyclohex-4-en-1.2-dicarbonsäure, Cyclohexen-1.2-dicarbonsäureanhydrid, 4-Methylcyclohexan-1.2-dicarbonsäure, 4-Methylcyclohexan-1.2-dicarbonsäureanhydrid, 4-Methylcyclohex-4-en-1.2-dicarbonsäure, 4-Methylcyclohex-4-en-1.2-dicarbonsäureanhydrid. Beispiele für aromatische Verbindungen sind: Phthalsäure, Phthalsäureanhydrid, Isophthalsäure, Therephthalsäure, Trimellitsäure, Trimesinsäure, Pyromellitsäure, Pyromellitsäureanhydrid oder Naphthalindicarbonsäuren. Bevorzugt eingesetzte Säuren sind Phthalsäure, Cyclohexandicarbonsäure und/oder Trimellitsäure und/oder deren Anhydride.

Im erfindungsgemäßen Verfahren werden bevorzugt verzweigte oder lineare aliphatische Alkohole mit 4 bis 13 C-Atomen eingesetzt. Die Alkohole sind bevorzugt einwertig und können sekundär oder primär sein.

Die eingesetzten Alkohole können aus verschiedenen Quellen stammen. Geeignete Einsatzstoffe sind beispielsweise Fettalkohole, Alkohole aus dem Alfolprozess oder Alkohole oder Alkoholgemische, die durch Hydrierung von gesättigten oder ungesättigten Aldehyden gewonnen wurden, insbesondere solchen, deren Synthese einen Hydroformylierungsschritt einschließt.

Alkohole, die im erfindungsgemäßen Verfahren eingesetzt werden, sind beispielsweise n-Butanol, Isobutanol, n-Octanol (1), n-Octanol(2), 2-Ethylhexanol, Nonanole, Decylalkohole oder Tridecanole hergestellt durch Hydroformylierung von Olefinen oder Aldolkondensation von Aldehyden und anschließende Hydrierung. Die Alkohole können als reine Verbindung, als Gemisch isomerer Verbindungen oder als Gemisch von Verbindungen mit unterschiedlicher C-Zahl eingesetzt werden.

Bevorzugte Einsatzalkohole sind Gemische isomerer Octanole, Nonanole oder Tridecanole, wobei die letzteren aus den entsprechenden Butenoligomeren, insbesondere Oligomeren von linearen Butenen, durch Hydroformylierung und anschließender Hydrierung gewonnen werden können. Die Herstellung der Butenoligomeren kann im Prinzip nach drei Verfahren durchgeführt werden. Die sauer katalysierte Oligomerisierung, bei der technisch z. B. Zeolithe oder Phosphorsäure auf Trägern eingesetzt werden, liefert die verzweigtesten Oligomere. Bei Einsatz von linearen Butenen entsteht beispielsweise eine C₈-Fraktion, die im Wesentlichen aus Dimethylhexenen besteht (WO 92/13818). Ein ebenfalls weltweit ausgeübtes Verfahren ist die Oligomerisierung mit löslichen Ni-Komplexen, bekannt als DIMERSOL-Verfahren (B. Cornils, W. A. Herrmann, Applied Homogenous Catalysis with Organometallic Compounds, Seite 261-263, Verlag Chemie 1996). Weiterhin wird die Oligomerisierung an Nickel-FestbettKatalysatoren ausgeübt, wie beispielsweise der OCTOL-Process (Hydrocarbon Proess., Int. Ed. (1986) 65 (2. Sect. 1), Seite 31-33).

Ganz besonders bevorzugte Einsatzstoffe für die erfindungsgemäße Veresterung sind Gemische isomerer Nonanole oder Gemische isomerer Tridecanole, die durch Oligomerisierung linearer Butene zu C₈-Olefinen und C₁₂-Olefinen nach dem Octol-Prozess, mit anschließender Hydroformylierung und Hydrierung hergestellt werden.

Die Veresterung wird in einem Reaktionsgefäß durchgeführt, in dem der Reaktionsansatz mit Hilfe eines Rührers oder einer Umlaufpumpe intensiv vermischt werden kann. Die Edukte und der Katalysator können gleichzeitig oder hintereinander in den Reaktor eingefüllt werden. Ist ein Einsatzstoff bei der Einfülltemperatur fest, ist es zweckmäßig, die flüssige Einsatzkomponente vorzulegen. Feste Einsatzstoffe können als Pulver, Granulat, Kristallisat oder Schmelze eingespeist werden. Um die Chargenzeit zu verkürzen, ist es ratsam, während des Einfüllens mit dem Aufheizen zu beginnen. Der Katalysator kann in reiner Form oder als Lösung, bevorzugt gelöst in einem der Einsatzstoffe, zu Beginn oder erst nach Erreichen der Reaktionstemperatur eingebracht werden. Carbonsäureanhydride reagieren häufig mit Alkoholen bereits autokatalytisch, d. h. unkatalysiert zu den entsprechenden Estercarbonsäuren (Halbestern), beispielsweise Phthalsäureanhydrid zum Phthalsäuremonoester. Daher ist ein Katalysator häufig erst nach dem ersten Reaktionsschritt erforderlich.

Mit Hilfe des erfindungsgemäßen Verfahrens können Estergemische aufgearbeitet werden, die durch autokatalysierte oder fremdkatalysierte Reaktion von Carbonsäuren mit Alkoholen entstanden sind.

Als Veresterungskatalysatoren können im erfindungsgemäßen Verfahren Lewis- oder Brøndstedtsäuren oder metallorganische Stoffe, die nicht unbedingt als Säure wirken müssen, eingesetzt werden. Bevorzugte Veresterungskatalysatoren sind Alkoholate, Carbonsäuresalze oder Chelatverbindungen von Titan oder Zirkonium, wobei das Katalysatormolekül ein oder mehrere Metallatome enthalten kann. Insbesondere werden Tetra(isopropyl)orthotitanat und Tetra(butyl)orthotitanat eingesetzt.

Organische Titan- oder Zirconiumkatalysatoren werden zweckmäßig erst bei Temperaturen größer oder gleich 140 °C zugesetzt. Die Katalysatorkonzentration hängt von der Art des Katalysators ab. Bei den bevorzugt eingesetzten Titanverbindungen beträgt die Konzentration des Katalysators 0,005 bis 1,0 Massen-% bezogen auf das Reaktionsgemisch, insbesondere 0,01 bis 0,5 Massen-%, ganz besonders 0,01 bis 0,1 Massen-%.

Der umzusetzende Alkohol, der als Schleppmittel dient, wird im stöchiometrischen Überschuss, bevorzugt 5 bis 50 %, besonders bevorzugt 10 bis 30 % der stöchiometrisch notwendigen Menge eingesetzt.

Die Reaktionstemperaturen liegen bei Verwendung von Titankatalysatoren zwischen 160 °C und 270 °C, vorzugsweise bei 180 bis 250 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Normaldruck oder leichtem Überdruck und bei höher siedenden Alkoholen bei verminderten Druck durchgeführt. Beispielsweise wird bei der Umsetzung von Phthalsäureanhydrid mit einem Gemisch isomerer Nonanole in einem Temperaturbereich von 170 °C bis 250 °C im Druckbereich von 1 bar bis 10 mbar gearbeitet.

Es ist möglich, das während der Veresterungsreaktion entstandene Reaktionswasser durch azeotrope Destillation während der laufenden Veresterungsreaktion zu entfernen. Durch diese optionale Maßnahmen wird nicht nur das Reaktionswasser, sondern auch ein Teil des Alkohols entfernt.

Die in die Reaktion zurückzuführende Flüssigkeitsmenge kann teilweise oder vollständig aus dem Einsatzalkohol bestehen. Dieser Alkohol kann durch Aufarbeitung des azeotropen Destillats gewonnen werden. Es ist auch möglich, die Aufarbeitung zu einem späteren Zeitpunkt durchzuführen und die entfernte Flüssigkeitsmenge ganz oder teilweise durch frischen Alkohol, d. h. aus einem im Vorratsgefäß bereitstehenden Alkohol zu ersetzen.

Während der Reaktion wird ein Alkohol-Wasser-Gemisch als Azeotrop aus der Reaktionsmischung abdestilliert. Die Brüden verlassen z. B. über eine kurze Kolonne (Einbauten oder Füllkörper; 1 bis 5, vorzugsweise 1 bis 3 theoretische Böden) das Reaktionsgefäß und werden kondensiert. Das Kondensat kann in eine wässrige und in eine alkoholische Phase getrennt werden; dies kann wiederum eine Kühlung erforderlich machen. Die wässrige Phase wird abgetrennt und kann, gegebenenfalls nach Aufarbeitung, verworfen oder als Strippwasser bei der Nachbehandlung des Esters verwendet werden.

Die alkoholische Phase, die nach Trennung des azeotropen Destillat ausfällt, kann teilweise oder vollständig in das Reaktionsgefäß zurückgeführt werden. In der Praxis hat sich eine standgeregelte Füllstandskontrolle der Reaktion zur Zufuhr des Alkohols bewährt.

Es ist möglich, die durch die azeotrope Destillation entfernte Flüssigkeitsmenge vollständig oder teilweise zu ersetzen, in dem die abgetrennte Flüssigkeit in eine Alkohol- und Wasserphase getrennt wird und die Alkoholphase in die Veresterungsreaktion zurückgeführt wird.

Optional kann frischer Alkohol zur abgetrennten Alkoholphase, die in die Reaktion zurückgeführt wird, beigefügt werden.

Für die Zufuhr des Alkohols in die Veresterungsreaktion gibt es verschiedene Möglichkeiten. Der Alkohol kann beispielsweise als Rückfluss auf die Kolonne gegeben werden. Eine andere Möglichkeit ist, den Alkohol, gegebenenfalls nach Aufheizung, in das flüssige Reaktionsgemisch zu pumpen. Durch die Abtrennung des Reaktionswasser sinkt das Reaktionsvolumen in der Apparatur. Es ist jedoch vorteilhaft, wie in DE 100 43 545.9 beschrieben (kürzere Reaktionszeit) während der Reaktion so viel Alkohol nachzuspeisen, wie dem Volumen des abgetrennten Destillats (Wasser und gegebenenfalls Alkohol) entspricht, sodass der Füllstand im Reaktionsgefäß konstant bleibt. Durch die Vergrößerung des Alkoholüberschusses wird das Gleichgewicht zu Gunsten der Vollester verschoben.

Nach Beendigung der Reaktion enthält das Reaktionsgemisch, das im Wesentlichen aus Vollester (Zielprodukt) und überschüssigem Alkohol besteht, neben dem Katalysator und/oder dessen Folgeprodukte geringe Mengen an Estercarbonsäure(n) (Halbester) und/oder nicht umgesetzter Carbonsäure und gegebenenfalls Zersetzungsprodukte des Alkohols wie beispielsweise Olefine.

Wird im erfindungsgemäßen Verfahren z. B. Phthalsäureanhydrid mit Isononanol oder 2-Ethylhexanol umgesetzt, so erfolgt die Veresterung bei einer Temperatur von 180 bis 250 °C, optional unter ständiger Wasserentfernung durch Azeotropdestillation. In einer Ausführungsform erfolgt dies so lange, bis die Säurezahl unter 0,5 gesunken ist. Dann werden unter Verringerung des Druckes, beispielsweise bis auf 15 mbar, der überschüssige Alkohol, geringe Mengen an Reaktionswasser und gegebenenfalls andere Leichtsieder abdestilliert. Durch die dabei fast vollständige Entfernung des Reaktionswassers steigt der Säureumsatz und die Säurezahl sinkt noch deutlich weiter ab. Während des Abdestillieren kann auf Zufuhr von Energie verzichtet werden, um die Temperatur des Veresterungsgemisches vor der Neutralisation zu senken. Werden andere Säuren bzw. Alkohole eingesetzt, müssen die Reaktionstemperaturen, -drücke und -zeiten entsprechend angepasst werden. So erfolgt die Herstellung von Dibutylphthalat mit Schwefelsäure als Katalysator bei 140 bis 180 °C.

Die Abtrennung des Überschußalkohols kann zunächst durch Vakuumdestillation, ohne weitere Energiezufuhr bis zu einer Alkoholkonzentration < 1 % im Reaktionsgemisch erfolgen. Anschließend können die restlichen Leichtsieder durch eine Wasserdampfdestillation bei ≥ 190 °C unter Energiezufuhr (1 h) und dann unter Abkühlung (Wasserdampfdestillation ohne Energiezufuhr) auf 150 °C entfernt werden. Mit Beginn der Wasserdampfdestillation erfolgt parallel auch die Neutralisation.
Die Wasserdampfdestillation bewirkt neben der Abtrennung der restlichen Alkoholspuren und sonstiger Leichtsieder auch die Zersetzung des Katalysators zu einem leicht filtrierbaren Feststoff

Der Destillationsdruck während der Wasserdampfdestillation liegt zwischen 500 und 50 mbar, insbesondere zwischen 150 und 80 mbar.

Die Wasserdampfdestillation erfolgt im Temperaturbereich von 230 °C bis 130 °C, insbesondere im Bereich von 230 bis 150 °C. Während der Wasserdampfdestillation wird die Temperatur im Allgemeinen mit einer Geschwindigkeit von 5 bis 40 °C/h gesenkt. Die Temperatur kann über den Energiegehalt des eingebrachten Wassers oder Dampfes sowie durch direkten Wärmeaustausch (Wärmetauscher) reguliert werden.

Bei der Wasserdampfdestillation werden je Tonne Rohester 5 bis 40 kg/h Wasser (flüssig und/oder dampfförmig), insbesondere 10 bis 30 kg/h eingesetzt. Der spezifische Wasserverbrauch (kg/(t*h)) kann während der Wasserdampfdestillation konstant oder verschieden sein.

Während der Wasserdampfdestillation, im Temperaturintervall von 220 bis 160 °C, werden zur Neutralisation der saueren Stoffe, wie Carbonsäuren, Estercarbonsäuren oder gegebenenfalls der sauren Katalysatoren, basisch wirkende Stoffe z. B. Verbindungen der Alkali- oder Erdalkalimetalle zugesetzt. Es können auch Gemische verschiedener Basen verwendet werden. Das Neutralisationsmittel kann in fester Form oder bevorzugt als Lösung, insbesondere als wässrige Lösung eingesetzt werden. Wenn eine wässrige Lösung einer Base eingesetzt wird, liegt der Gehalt der basischen Verbindung zwischen 1 und 30 Massen-%, bevorzugt zwischen 5 und 30 %, besonders bevorzugt zwischen 20 bis 25 %. Ein bevorzugtes Neutralisationsmittel ist Natronlauge.

Die Zugabe der Base erfolgt bevorzugt ab einer Säurezahl des Rohesters von kleiner 1, bevorzugt 0,5, besonders bevorzgut kleiner 0,3. Die Säurezahl ist durch den Verbrauch von KOH bei der Titration des Reaktionsgemisches gegen Phenolphthalein in mg KOH/g Produkt definiert.

In einer weiteren Verfahrensvariante werden 0,005 bis 0,008 Val (5 - 8 mVal) Lauge bezogen auf ein kg Rohestergemisch zugesetzt. Dies bedeutet beispielsweise den Zusatz von 0,8 bis 1,28 kg 25 %-iger Natronlauge je Tonne Rohestergemisch.

Die Lauge kann zum Rohestergemisch zugetropft werden. Im erfindungsgemäßen Verfahren wird jedoch bevorzugt die Lauge bei verminderten Druck über Düsen am und/oder in der Nähe des Reaktorbodens in das Rohestergemisch eingebracht. In einer weiteren Ausführungsform erfolgt die Zudosierung der Lauge durch Düsen am und/oder in der Nähe des Rührkörpers.

Die Lauge wird unten in das Reaktionsgemisch eingedüst. Durch die hohe Temperatur verdampft das Wasser. Die Dampfblasen sorgen neben dem Rührer für eine gute Verteilung der Lauge. Die Düsen (z. B. 4 Stück) sollen örtlich weit voneinander entfernt sein, so dass die Lauge gleichmäßig im Gemisch verteilt wird.

Dabei beträgt die spezifische Zudosiergeschwindigkeit der Lauge in der Regel nur 1 bis 5, insbesondere nur 2 bis 3 Liter je Stunde und je Tonne Rohestergemisch. Durch dieses Vorgehen wird die Laugekonzentration im Gemisch schnell verteilt und somit das Ausmaß von Nebenreaktionen, wie beispielsweise die Verseifung von Ester, gering gehalten.

Im Allgemeinen liegt der Wassergehalt im Estergemisch direkt nach der Wasserdampfdestillation deutlich unter 0,1 Massen-% .

Optional kann nach der Wasserdampfdestillation das Estergemisch bei einer Temperatur von 180 bis 130 °C , insbesondere 180 bis 150 °C bei einem Druck von 50 bis 20 mbar, insbesondere 20 bis 10 mbar weiter entwässert werden, bis der Wassergehalt unter 0,05 Massen-% gesunken ist. Dazu werden in der Regel weniger als 10 Minuten Trocknungszeit benötigt.

Der Ester kann ohne weitere Trocknung mit Inertgas, gegebenenfalls nach Zusatz eines Filterhilfsmittel, filtriert werden. Optional können die Feststoffe durch Zentrifugieren abgetrennt werden. Die Stofftrennung erfolgt im Temperaturbereich 150 °C bis 20 °C.

Das erfindungsgemäße Verfahren kann in einem Behälter oder in mehreren hintereinander geschalteten Gefäßen, jeweils bevorzugt Rührreaktoren, durchgeführt werden. So kann beispielsweise die Veresterung und die Aufarbeitung in verschiedenen Gefäßen erfolgen.

In einer besonderen Variante des erfindungsgemäßen Verfahrens ist es möglich, die Veresterungsreaktion nur teilweise, d. h. bis zum Partialester durchzuführen. Beispielsweise kann die Reaktion im Falle der Phthalsäure bis zum Monoester geführt werden. Dies ist z. B. bei Einsatz von festem Phthalsäureanhydrid (PSA) sinnvoll. Festes PSA wird in einem "Lösebehälter" unter Rühren bei 130 °C in Alkohol gelöst, wobei sich der flüssige Halbester bildet. Dieser kann mit weiterem Alkohol in den eigentlichen Veresterungsreaktor gepumpt werden. Es ist dann möglich, die Veresterungsreaktion des Partialesters zum Vollester im gleichen oder in einem anderen Reaktor weiter durchzuführen.

Die der Bildung des Partialesters folgende Veresterungsreaktion kann gemäß den erfindungsgemäßen Verfahren durchgeführt werden.

Die Ester aus mehrbasischen Carbonsäuren, wie beispielsweise Phthalsäure, und aus Alkoholen, finden weite Anwendung in Lackharzen, als Bestandteile von Anstrichmitteln und insbesondere als Weichmacher für Kunststoffe. Geeignete Weichmacher für PVC sind Diisononylphthalate und Dioctylphthalate. Weiterhin können die erfindungsgemäß hergestellten Ester als Komponente von Schmierstoffen Verwendung finden. Die Verwendung der erfindungsgemäß hergestellten Ester für diese Zwecke ist ebenfalls Gegenstand der Erfindung.

Der bei der Aufarbeitung abgetrennte Alkohol kann, gegebenenfalls nach Ausschleusung einer Teilmenge, für den nächsten Ansatz verwendet werden.

Die folgenden Beispiele sollen die Erfindung näher erläutern, ohne den Schutzbereich, wie in den Patentansprüchen definiert, zu beschränken.

### Beispiel 1: Herstellung eines Rohestergemisches

Der verwendete Veresterungsreaktor besteht aus einem Rührbehälter mit Heizschlange (40 bar Dampf), einem Trennsystem für die Reaktionswasser/Alkohol-Trennung und einer Rücklaufleitung für den Überschussalkohol. Die Apparatur wird vor Befüllung mit Stickstoff sauerstofffrei gespült.

### Einsatzmengen:

1000 kg Phthalsäureanhydrid (flüssig)
2430 kg Isononanol
1 kg Butyltitanat

Sobald die ersten 400 kg Isononanol im Reaktor vorgelegt worden waren, wurde mit dem Aufheizen begonnen. Phthalsäureanhydrid in flüssiger Form und die Restalkoholmenge (2 030 kg) wurden gleichzeitig eingespeist. Nachdem das Reaktionsgemisch eine Temperatur von 140 °C erreicht hatte, wurde der Titankatalysator zugegeben. Bei 170 °C begann das Gemisch zu sieden. Während der Veresterung wurde Wasser freigesetzt und als Isononanol-Azeotrop abdestilliert. Die Säurezahl der Reaktionsmischung sank von einem Startwert von 100 mg KOH/g bei Siedebeginn auf 10 mg KOH/g nach 150 Minuten, 1 mg KOH nach 290 Minuten und 0,5 mg KOH/g nach 330 Minuten. Der Anlagendruck war zu diesem Zeitpunkt stufenweise bis auf 300 mbar verringert worden, die Reaktionstemperatur betrug 220 °C, um den gewünschten Alkoholrücklauf zur effektiven Wasserabtrennung aufrechterhalten zu können. Der Alkoholgehalt im Reaktionsgemisch lag am Ende der Veresterung bei 6,5 Massen-%.

### Beispiel 2 (Vergleichsbeispiel für die Aufarbeitung)

Das nach Beispiel 1 hergestellte Rohestergemisch wurde bei 300 mbar und 220 °C unter Alkoholrücklauf durch Eindüsen von 13,2 kg 25 %iger Natronlauge innerhalb von 30Minuten neutralisiert. Die Säurezahl des Reaktionsgemisch sank dabei auf 0,03 mg KOH/g. Anschließend wurde der Überschußalkohol ohne weitere Energiezufuhr unter Vakuum abdestilliert. Die Destillation dauerte 40 Minuten. Der Alkoholgehalt der Mischung lag danach bei 0,51 Massen-%. Die Temperatur sank auf 200 °C ab. Die Säurezahl verschlechterte sich aufgrund der Verringerung des Reaktionsgemisches auf 0,035 mg KOH/g.

Nun wurden die übrigen Nachbehandlungsschritte durchgeführt (Wasserdampfdestillation bei 200 °C, Abkühlung auf 130° C, Filtration), wobei die Säurezahl der Mischung konstant blieb. Der Alkoholgehalt im Endprodukt lag bei 110 ppm, der Wassergehalt bei 0,04 Gew. %, die Farbzahl (APHA/Hazen) bei 20 mg Pt/l.

### Beispiel 3 (Aufarbeitung gemäß der Erfindung)

Aus dem nach Beispiel 1 hergestellten Rohestergemisch wurde im Vakuum, das stufenweise von 300 auf 20 mbar vermindert wurde, ohne Energiezufuhr der größte Teil des überschüssigen Alkohols in 40 Minuten abdestilliert, wobei die Temperatur auf 202 °C fiel. Die Säurezahl der Reaktionsmischung lag nun bei nur noch 0,12 mg KOH/g und es genügten 3,6 kg der 25%igen NaOH, die innerhalb von 9 Minuten zudosiert wurden, um den Ansatz auf eine Säurezahl von 0,03 mg KOH/g zu neutralisieren. Die weiteren Nachbehandlungsschritte erfolgten wie bei Beispiel 2. Die Säurezahl verschlechterte sich nicht mehr. Das Produkt wies nach der Filtration folgende Werte auf:
Säurezahl: 0,03 mg KOH/g
Wassergehalt: 0,04 Gew.%
Alkoholgehalt: 100 ppm
Farbzahl: 10 mg Pt/l

Aufgrund des deutlich geringeren NaOH-Bedarfs im Beispiel 3 im Vergleich zu Beispiel 2, verringerte sich der Zeitbedarf für die Neutralisation und Filtersalzanfall entsprechend der geringeren NaOH-Menge. Das wirkte sich positiv auf die Nachbehandlungszeit, den Einsatzstoff-Faktor für die Weichmacherproduktion, den Anfall an zu entsorgenden Filtersalzen und die Filtrationszeit aus, die um 25 % reduziert werden konnte.

## Patentansprüche

1. Verfahren zur Herstellung von Carbonsäureestern durch Reaktion von Di- oder Polycarbonsäuren oder deren Anhydriden mit Alkoholen, wobei der überschüssige Alkohol nach der Veresterungsreaktion entfernt, der so erhaltene Rohester durch Basenzugabe neutralisiert und anschließend filtriert wird,
**dadurch gekennzeichnet,**
**dass** der Alkohol durch mindestens eine Wasserdampfdestillation abgetrennt und die Basenzugabe während einer Wasserdampfdestillation erfolgt.

2. Verfahren nach Anspruch 1,
**dadurch gekennzeichnet,**
**dass** das Reaktionswasser durch azeotrope Destillation mit dem Alkohol während der Veresterungsreaktion entfernt wird.

3. Verfahren nach Anspruch 2,
**dadurch gekennzeichnet,**
**dass** die durch die azeotrope Destillation aus der Reaktion entfernte Flüssigkeitsmenge vollständig oder teilweise mit dem Alkohol wieder ergänzt wird.

4. Verfahren nach Anspruch 2 oder 3,
**dadurch gekennzeichnet,**
**dass** die durch die azeotrope Destillation abgetrennte Flüssigkeitsmenge vollständig oder teilweise ergänzt wird, indem die abgetrennte Flüssigkeit in eine Wasserphase und eine Alkoholphase getrennt wird und die Alkoholphase in die Veresterungsreaktion zurückgeführt wird.

5. Verfahren nach Anspruch 2 oder 3,
**dadurch gekennzeichnet,**
**dass** die durch azeotrope Destillation abgetrennte Flüssigkeitsmenge vollständig oder teilweise ergänzt wird, indem die abgetrennte Flüssigkeit in eine Wasserphase und eine Alkoholphase getrennt wird und die Alkoholphase, zusätzlich mit frischem Alkohol versetzt, in die Veresterungsreaktion zurückgeführt wird.

6. Verfahren nach Anspruch 2 oder 3,
**dadurch gekennzeichnet,**
**dass** die durch die azeotrope Destillation aus der Reaktion entfernte Flüssigkeitsmenge durch frischen Alkohol vollständig oder teilweise wieder ergänzt wird.

7. Verfahren nach einem der Ansprüche 1 bis 6,
**dadurch gekennzeichnet,**
**dass** die Basenzugabe bei einer Säurezahl des Rohesters von kleiner 1 erfolgt.

8. Verfahren nach einem der Ansprüche 1 bis 7,
**dadurch gekennzeichnet,**
**dass** die Abtrennung des Alkohols durch eine Vakuumdestillation und eine anschließende Wasserdampfdestillation erfolgt.

9. Verfahren nach einem der Ansprüche 1 bis 8,
**dadurch gekennzeichnet,**
**dass** die Basenzugabe durch Düsen an oder in der Nähe des Reaktorbodens erfolgt.

10. Verfahren nach einem der Ansprüche 1 bis 8,
**dadurch gekennzeichnet,**
**dass** die Basenzugabe durch Düsen an oder in der Nähe des Rührkörpers erfolgt.

11. Verfahren nach Anspruch 1 bis 10,
**dadurch gekennzeichnet,**
**dass** als Di- oder Polycarbonsäure Phthalsäure, 1.2-Cyclohexandicarbonsäure und/oder Trimellitsäure und/oder deren Anhydride eingesetzt werden.

12. Verfahren nach Anspruch 1 bis 11,
**dadurch gekennzeichnet,**
**dass** als Alkohol n-Butanol, Isobutanol, n-Octanol (1), n-Octanol (2), 2-Ethylhexanol, Nonanole, Decylalkohole oder Tridecanole eingesetzt werden.

13. Verfahren nach einem der Ansprüche 1 bis 12,
**dadurch gekennzeichnet,**
**dass** Di- oder Polycarbonsäuren vollständig verestert werden.

## Claims

1. A process for preparing a carboxylic acid ester by reacting a dicarboxylic or polycarboxylic acid or its anhydride with an alcohol, the excess alcohol being removed after the esterification reaction, the resultant crude ester being neutralized by adding base and then filtered, **characterized in that** the alcohol is separated off by at least one steam distillation and the base is added during a steam distillation.

2. A process according to claim 1, **characterized in that** the reaction water is removed together with the alcohol by azeotropic distillation during the esterification reaction.

3. A process according to claim 2, **characterized in that** the quantity of liquid removed from the reaction by the azeotropic distillation is completely or partially replaced by the alcohol.

4. A process according to claim 2 or 3, **characterized in that** the quantity of liquid separated off by the azeotropic distillation is completely or partially replaced by separating the liquid removed into a water phase and an alcohol phase and recycling the alcohol phase to the esterification reaction.

5. A process according to claim 2 or 3, **characterized in that** the quantity of liquid separated off by azeotropic distillation is completely or partially replaced by separating the liquid removed into a water phase and an alcohol phase and recycling the alcohol phase, additionally admixed with fresh alcohol, to the esterification reaction.

6. A process according to claim 2 or 3, **characterized in that** the quantity of liquid removed from the reaction by the azeotropic distillation is completely or partially replaced by fresh alcohol.

7. A process according to any one of claims 1 to 6, **characterized in that** the base is added at an acid number of the crude ester of less than 1.

8. A process according to any one of claims 1 to 7, **characterized in that** the alcohol is separated off by vacuum distillation and subsequent steam distillation.

9. A process according to any one of claims 1 to 8, **characterized in that** the base is added via nozzles on or near the reactor bottom.

10. A process according to any one of claims 1 to 8, **characterized in that** the base is added via nozzles on or near the agitator.

11. A process according to any of claims 1 to 10, **characterised in that** phthalic acid, 1,2-cyclohexanedicarboxylic acid and/or trimellitic acid and/or their anhydrides are used as dicarboxylic or polycarboxylic acid.

12. A process according to any of claims 1 to 11, **characterized in that** n-butanol, isobutanol, octan-1-ol, octan-2-ol, 2-ethylhexanol, a nonanol, a decyl alcohol or a tridecanol is used as alcohol.

13. A process according to any one of claims 1 to 12, **characterized in that** a dicarboxylic or polycarboxylic acid is completely esterified.

## Revendications

1. Procédé de préparation d'esters d'acides carboxyliques par réaction d'acides di- ou polycarboxyliques ou de leurs anhydrides avec des alcools, l'alcool en excès étant éliminé après la réaction d'estérification, l'ester brut ainsi obtenu étant neutralisé par addition de base et ensuite filtré,
**caractérisé en ce que**
l'alcool est séparé par au moins une distillation de vapeur d'eau et l'addition de base a lieu pendant une distillation de vapeur d'eau.

2. Procédé selon la revendication 1,
**caractérisé en ce que**
l'eau de réaction est éliminée par distillation azéotropique avec l'alcool pendant la réaction d'estérification.

3. Procédé selon la revendication 2,
**caractérisé en ce que**
la quantité de liquide éliminée de la réaction par la distillation azéotropique est remplacée entièrement ou partiellement par l'alcool.

4. Procédé selon la revendication 2 ou 3,
**caractérisé en ce que**
la quantité de liquide séparée par la distillation azéotropique est remplacée entièrement ou partiellement, en séparant le liquide séparé en une phase eau et une phase alcool et en renvoyant la phase alcool dans la réaction d'estérification.

5. Procédé selon la revendication 2 ou 3,
**caractérisé en ce que**
la quantité de liquide séparée par la distillation azéotropique est remplacée entièrement ou partiellement, en séparant le liquide séparé en une phase eau et une phase alcool et en renvoyant la phase alcool, à laquelle a été ajouté de l'alcool frais, dans la réaction d'estérification.

6. Procédé selon la revendication 2 ou 3,
**caractérisé en ce que**
la quantité de liquide éliminée de la réaction par la distillation azéotropique est remplacée entièrement ou partiellement par de l'alcool frais.

7. Procédé selon une des revendications 1 à 6,
**caractérisé en ce que**
l'addition de base a lieu pour un indice d'acide de l'ester brut inférieur à 1.

8. Procédé selon une des revendications 1 à 7,
**caractérisé en ce que**
la séparation de l'alcool a lieu au moyen d'une distillation sous vide suivie d'une distillation de vapeur d'eau.

9. Procédé selon une des revendications 1 à 8,
**caractérisé en ce que**
l'addition de base a lieu au moyen de buses sur le fond du réacteur ou à proximité de celui-ci.

10. Procédé selon une des revendications 1 à 8,
**caractérisé en ce que**
l'addition de base a lieu au moyen de buses sur le corps d'agitation ou à proximité de celui-ci.

11. Procédé selon une des revendications 1 à 10,
**caractérisé en ce que**
comme acide di- ou polycarboxylique, on utilise l'acide phthalique, l'acide 1,2-cyclohexanedicarboxylique et/ou l'acide trimellitique et/ou leurs anhydrides.

12. Procédé selon une des revendications 1 à 11,
**caractérisé en ce que**
comme alcool, on utilise le n-butanol, l'isobutanol, le n-octanol (1), le n-octanol (2), le 2-éthylhexanol, les nonanols, les alcool décyliques ou les tridécanols.

13. Procédé selon une des revendications 1 à 12,
**caractérisé en ce que**
les acides di- et polycarboxyliques sont entièrement estérifiés.
